Europäisches Patentamt

European Patent Office

Office européen des brevets

(19)

(11) Veröffentlichungsnummer : **0 229 203**
**B1**

# (12) EUROPÄISCHE PATENTSCHRIFT

(45) Veröffentlichungstag der Patentschrift :
27.07.88

(21) Anmeldenummer : 86100314.3

(22) Anmeldetag : 11.01.86

(51) Int. Cl.⁴ : **A 47 K 7/04, A 61 B 19/00**

(54) **Waschautomat zur Reinigung von Gegenständen.**

(43) Veröffentlichungstag der Anmeldung :
22.07.87 Patentblatt 87/30

(45) Bekanntmachung des Hinweises auf die Patenterteilung : 27.07.88 Patentblatt 88/30

(84) Benannte Vertragsstaaten :
AT BE CH DE FR GB IT LI LU NL SE

(56) Entgegenhaltungen :
FR-A- 1 382 553
US-A- 2 424 509
US-A- 2 632 906
US-A- 3 940 819

(73) Patentinhaber : Vetter, Helmut
Marienplatz 81
D-7980 Ravensburg (DE)

(72) Erfinder : Vetter, Helmut
Marienplatz 81
D-7980 Ravensburg (DE)

(74) Vertreter : Fay, Hermann, Dipl.-Phys. Dr. et al
Ensingerstrasse 21 Postfach 1767
D-7900 Ulm (Donau) (DE)

## Beschreibung

Die Erfindung betrifft einen Waschautomaten zur Reinigung von Gegenständen, insbes. auch von Händen, mit einem im wesentlichen allseits geschlossenen Gehäuse mit zumindest einer Öffnung zur Einführung des zu reinigenden Gegenstands, ferner mit einer oder mehreren, im Innern des Gehäuses angeordneten, den Gegenstand mit flüssigen und/oder gasförmigen Reinigungsmedien beaufschlagenden, entsprechend ausrichtbaren Düsen, sowie mit einer ebenfalls im Innern des Gehäuses angeordneten, auf Form und Lage des Gegenstandes im Gehäuse ansprechenden Kontrollvorrichtung, die ein Freigabesignal für den Reinigungsvorgang erzeugt, wenn der Gegenstand die für den Reinigungsvorgang vorgesehene Form bzw. Gestalt aufweist und/oder vorgeschriebene Lage bzw. Ausrichtung im Gehäuse einnimmt.

Ein Waschautomat dieser Art ist aus der nicht vorveröffentlichten europ. Patentanmeldung 85 104 320 bekannt und dazu vorgesehen, eine sorgfältige und vollständige Reinigung von Gegenständen, insbes. aber auch von Händen beim Übergang von der üblichen verunreinigten Umgebung in einen Reinraum zu gewährleisten. Solche Reinräume bestehen in zahlreichen industriellen Produktionsbereichen wie auch im medizinischen Bereich und stellen erhebliche Reinheitsanforderungen hinsichtlich Partikelgehalt bzw. Verkeimungsniveau. Um das Einschleppen von Verunreinigungen oder Erregern zu vermindern, gibt es beispielsweise staubabweisende Arbeitskleidung. Ein besonderes Problem stellt jedoch die Reinigung der mit den reinzuhaltenden Erzeugnissen in Berührung kommenden Hände der Arbeitskräfte dar, zumal sie häufig unsachgemäß und oft auch nachlässig durchgeführt wird. In der pharmazeutischen Produktion, in Kliniken, in der Lebensmittelverarbeitung und in ähnlichen Bereichen können dadurch Verunreinigungen in die Produktion oder auf reine Oberflächen übertragen werden. Eine mangelhafte Reinigung der Hände, die in der Medizin und Pharmazie zu Infektionsgefahren und Produktionsausfällen führen kann, ist in der Praxis nur schwer nachprüfbar und daher kaum abzustellen.

Der in der europ. Patentanmeldung 85 104 320 beschriebene Waschautomat sorgt daher durch Automatisierung des Reinigungsvorgangs der Hände dafür, daß der Reinigungsprozeß stets in der vorgeschriebenen, den jeweiligen Gegebenenheiten angepaßten Weise abläuft, so daß ein reproduzierbares Reinigungsergebnis erreicht wird. Entscheidend ist dabei, daß die Hände während des Reinigungsvorgangs in einer bestimmten Lage im Waschautomaten angeordnet sind, so daß die gesamte Handoberfläche vollständig von dem Reinigungsvorgang erfaßt wird. Hierzu gehört beispielsweise auch, daß die Finger während des Reinigungsvorgangs gespreizt sind, so daß die Fingerzwischenräume ebenso der Reinigung unterzogen werden. Die dazu vorgesehene Kontrollvorrichtung besteht entweder aus mechanisch arbeitenden Tastelementen oder aus einer optoelektronischen Abtasteinrichtung, die den zu reinigenden Gegenstand linien- und flächenhaft abtastet und das ermittelte Bildinformationssignal einer Bewertungseinrichtung zugeführt. Beide Ausführungsformen haben sich an sich bewährt, sind jedoch in ihrer Realisierung verhältnismäßig aufwendig. Bei der mechanischen Abtastung muß für eine sorgfältige Abdichtung der beweglichen Teile gegenüber den Reinigungsflüssigkeiten gesorgt werden, während die bildhafte optoelektronische Abtastung einen erheblichen Aufwand in der Signalverarbeitung und -aufbereitung erfordert.

Ferner ist aus der FR-A-1 382 553 ein Waschgerät zur Reinigung von Händen mit einem im wesentlichen allseits geschlossenen Gehäuse mit zumindest einer Öffnung zur Einführung der Hände und ferner mit im Innern des Gehäuses angeordneten, die Hände mit flüssigen Reinigungsmedien beaufschlagenden Düsen bekannt.

Der Erfindung liegt die Aufgabe zugrunde, einen Waschautomat gemäß der nicht vorveröffentlichten europ. Patentanmeldung 85 104 320 so auszubilden, daß bei der Reinigung von Händen die Überwachung der vorgeschriebenen Fingerpositionierung auf einfache und zuverlässige Weise möglich ist.

Diese Aufgabe wird nach der Erfindung dadurch gelöst, daß zur Positionierung und zur anschließenden Erfassung der Lage der Finger einer zu reinigenden Hand die Kontrollvorrichtung für jeden der Finger eine eigene Auflagefläche aufweist, wobei die einzelnen Auflageflächen in den anatomischen Gegebenheiten entsprechender gegenseitiger Ausrichtung angeordnet sind und ihr Abstand so bemessen ist, daß die mit ihren Kuppen auf den Auflageflächen aufliegenden Finger gespreizt sind und mit ihren Kuppen die Auflagefläche abdecken, daß ferner jede der Auflageflächen mit einem optoelektronischen Sensor versehen ist, der bei nicht abgedeckter Auflagefläche von zumindest einer Strahlungsquelle beleuchtet ist und daß aus den Ausgangssignalen der Sensoren bei entsprechend dem Reinigungsvorgang vorgeschriebener Abdeckung das Freigabesignal erzeugt wird.

Der durch die Erfindung erreichte Fortschritt besteht im wesentlichen darin, daß die Lageerfassung der Hand einerseits optoelektronisch vorgenommen wird, wodurch keinerlei mechanisch zu betätigende Tastelemente notwendig sind und daher keine Abdichtungsprobleme auftreten können. Andererseits erfolgt auch keine rasterartige bildhafte Abtastung, die zu einem hohen Verarbeitungsaufwand bei der Auswertung führen würde. Die Abtastung der vorgeschriebenen Lage der zu reinigenden Hand erfolgt vielmehr in der Art zahlreicher Lichtschranken, wodurch die genannten mechanischen Probleme sowie eine aufwendige Auswertung von Bildsignalen entfallen.

In bevorzugter Ausführungsform der Erfindung ist jede der Auflageflächen jeweils von der Stirnfläche eines vertikal angeordneten Auflagekörpers gebildet. Diese Auflagekörper sorgen auch für einen entsprechenden Abstand der Handinnenfläche vom Gehäuseboden des Waschautomaten, so daß die Reinigung auch von der Unterseite her durch dort angebrachte Düsen ohne weiteres möglich ist. Der Auflagekörper weist dabei zweckmäßigerweise zylindrische Gestalt auf und ist in einer Bohrung eines die Auflagekörper haltenden, horizontal verlaufenden Verbindungsstegs angeordnet.

Die Auflagefläche kann an sich beliebige Oberflächengestalt aufweisen, so lange nur dafür gesorgt ist, daß eine Reinigung der Finger auch im der Auflagefläche anliegenden Bereich gewährleistet ist. In bevorzugter Ausführungsform der Erfindung ist daher die Auflagefläche konvex geformt, wobei in deren Scheitelbereich der Sensor kuppelförmig hervorsteht. Dadurch liegt die Fingerkuppe im wesentlichen lediglich punktförmig dem Sensor an, so daß praktisch keine die Reinigung behindernde Abdeckung erfolgt. Um die Reinigungswirkung in diesem Bereich zusätzlich zu erhöhen, empfiehlt es sich, daß in der Auflagefläche zumindest eine der Düsen zur Abgabe des Reinigungsmediums mündet. Dadurch werden auch durch den Auflagekörper verursachte Abschattungseffekte vermieden. Dabei ist es weiter vorteilhaft, wenn in der Auflagefläche zwei sich diametral gegenüberstehende Düsen münden.

Eine weitere wirksame Steigerung der Reinigungswirkung im Bereich der Auflagefläche wird dadurch erreicht, daß der Düsenkanal in Richtung zu seiner Mündungsöffnung schräg zur Zylinderachse des Auflagekörpers hin gerichtet verläuft, so daß die Reinigungsflüssigkeit etwa beim Auflagepunkt auf die Fingerkuppe auftrifft.

Um den Sensor einfach montieren und gegebenenfalls auch austauschen zu können, ist der Auflagekörper zweckmäßigerweise im wesentlichen quer zu seiner Achse zweigeteilt, wobei der obere, die Auflagefläche tragende Teil an seinem der Auflagefläche abgewandten unteren Rand einen Ringkragen aufweist, der ein am unteren Teil des Auflagekörpers koaxial vorstehendes Zylinderstück dichtend umgreift, wobei im Zylinderstück eine Axialbohrung für die elektrische Zuleitung zum Sensor vorgesehen ist und im Ringkragen axial verlaufende Verbindungskanäle an die Düsenkanäle anschließen und an der Stirnseite des Ringkragens mit einer oder mehreren Zuführleitungen am unteren Teil des Auflagekörpers in Verbindung stehen. Auf diese Weise sind sowohl die elektrischen Zuleitungen wie auch die Düsenkanäle ohne weiteres zugänglich, dennoch sind die beiden Systeme vollständig voneinander getrennt.

In einer weiter bevorzugten Ausführungsform der Erfindung sind jeweils etwa mittig zwischen den Auflageflächen weitere optoelektronische Sensoren vorgesehen. Durch Registrierung eines Lichteinfalls durch diese Sensoren kann beispielsweise sicher gestellt werden, daß die Finger sich tatsächlich in einer gespreizten Stellung befinden. Durch den Nachweis des Lichteinfalls über diese Sensoren ist im übrigen nachprüfbar, daß die Strahlungsquelle intakt ist und ordnungsgemäß arbeitet. Um die Fingerzwischenräume näher zur Handwurzel hin zu erfassen, kann es sich empfehlen, daß die die Fingerzwischenräume abtastenden Sensoren an dem Verbindungssteg angeordnet und gegenüber der Verbindungslinie der in den Auflageflächen vorgesehenen Sensoren in Richtung zur Handfläche hin verlagert sind.

Im folgenden wird die Erfindung an einem in der Zeichnung dargestellten Ausführungsbeispiel näher erläutert ; es zeigen :

Fig. 1 ein Handwaschgerät nach der Erfindung in schematischer Darstellung,

Fig. 2 eine Draufsicht auf die Kontrollvorrichtung des Waschautomaten mit aufgelegter, gestrichelt angedeuteter Hand,

Fig. 3 einen Auflagekörper in einer Schnittdarstellung,

Fig. 4 eine schematische Seitenansicht des Gegenstands nach Fig. 1.

Der in der Zeichnung dargestellte Waschautomat ist an sich allgemein zur Reinigung von Gegenständen vorgesehen, dient jedoch in der dargestellten Weise insbes. zur Reinigung von Händen. Dazu weist er ein im wesentlichen allseits geschlossenes Gehäuse 1 auf, das mit zwei Öffnungen 2 versehen ist, durch die die zu reinigenden Hände ins Innere des Waschautomaten eingeführt werden. Im Inneren des Gehäuses 1 sind mehrere Düsen 3 zur Abgabe von flüssigen und gegebenenfalls auch gasförmigen Reinigungsmedien angeordnet. Die Düsen 3 sind im einzelnen so ausgerichtet bzw. ausrichtbar, daß alle zu reinigenden Flächen der Hände erfaßt werden.

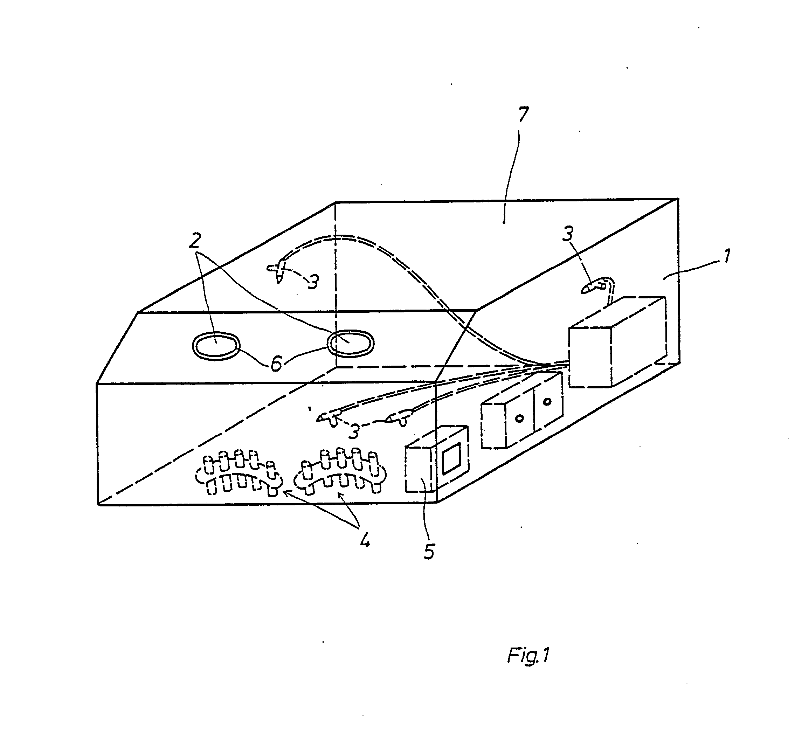

Im Inneren des Gehäuses 1 ist ferner eine in der Fig. 1 nur schematisch angedeutete Kontrollvorrichtung 4 angeordnet, die auf die Form und insbes. die Lage der zu reinigenden Hände im Gehäuse 1 anspricht. Sofern die Lage mit der für die Reinigung vorgesehenen übereinstimmt, wird der Reinigungsvorgang durch eine Steuereinrichtung 5 freigegeben, die im übrigen auch den verfahrensmäßigen und/oder zeitlichen Ablauf des Reinigungsvorgangs steuert und überwacht.

Die Öffnung 2 ist mit einer randseitig angeschlossenen elastischen Manschette 6 zur Abdichtung gegen das Handgelenk bzw. den Unterarm versehen. Dadurch wird verhindert, daß Reinigungsflüssigkeit aus dem Inneren des Gehäuses 1 nach außen gelangen kann. Ebenso wird das Eindringen von Staub und Keimen in das Gehäuse 1 während des Reinigungsvorgangs unterbunden. Dabei kann die Manschette 6 in in der Zeichnung nicht dargestellter Weise einen ringförmigen, im wesentlichen in Umfangsrichtung der Öffnung 2 verlaufenden und zur dichten Anlage am Arm mit Druck beaufschlagbaren Hohlraum aufweisen. Auf diese Weise ist ein leichtes Einführen der Hände in den Waschautomaten möglich, da die Manschette 6 sich dem Arm erst zu Beginn des Reinigungsvorgangs dichtend anlegt.

Das Gehäuse 1 kann in ebenfalls nicht näher dargestellter Weise als druckdichtschließendes und druckbeaufschlagbares Druckgefäß ausgebildet sein, um bedarfsweise eine thermisch antimikrobielle Behandlung des Innenraums durchführen zu können. Das Gehäuse[1] besteht aus einem gegen Reinigungsmedien resistenten und inerten Material wie beispielsweise Edelstahl, wobei die Oberfläche 7 des Gehäuses 1 zur Beobachtung des Reinigungsvorgangs von durchsichtigem Material wie beispielsweise Makrolon gebildet sein kann.

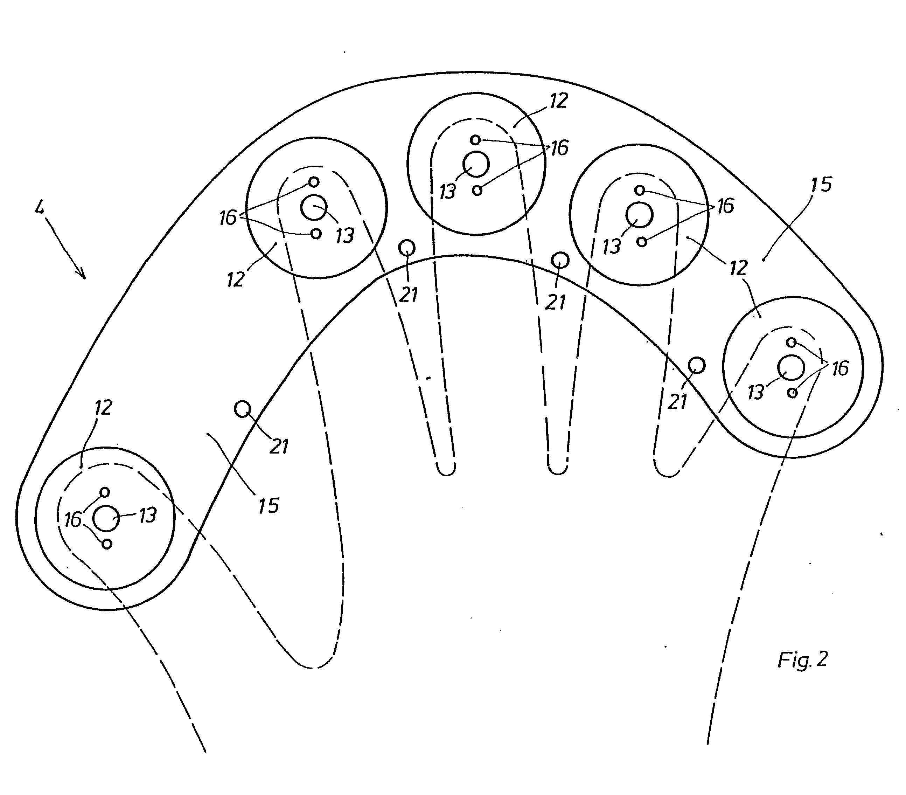

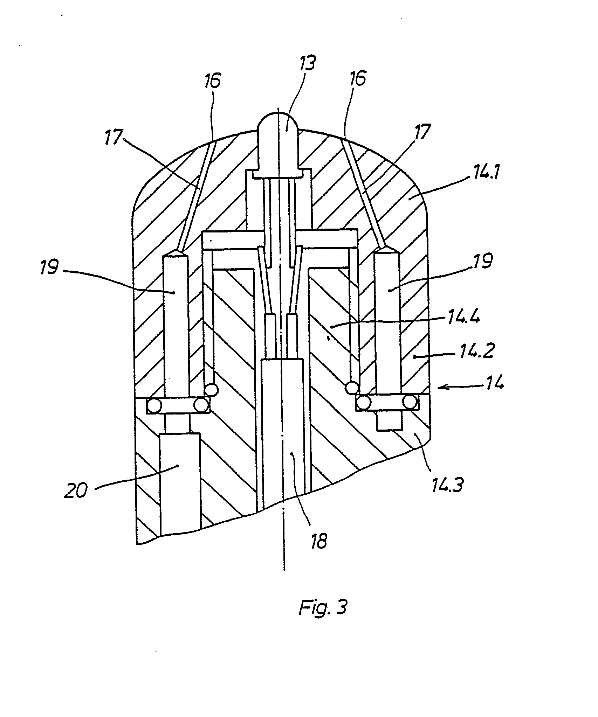

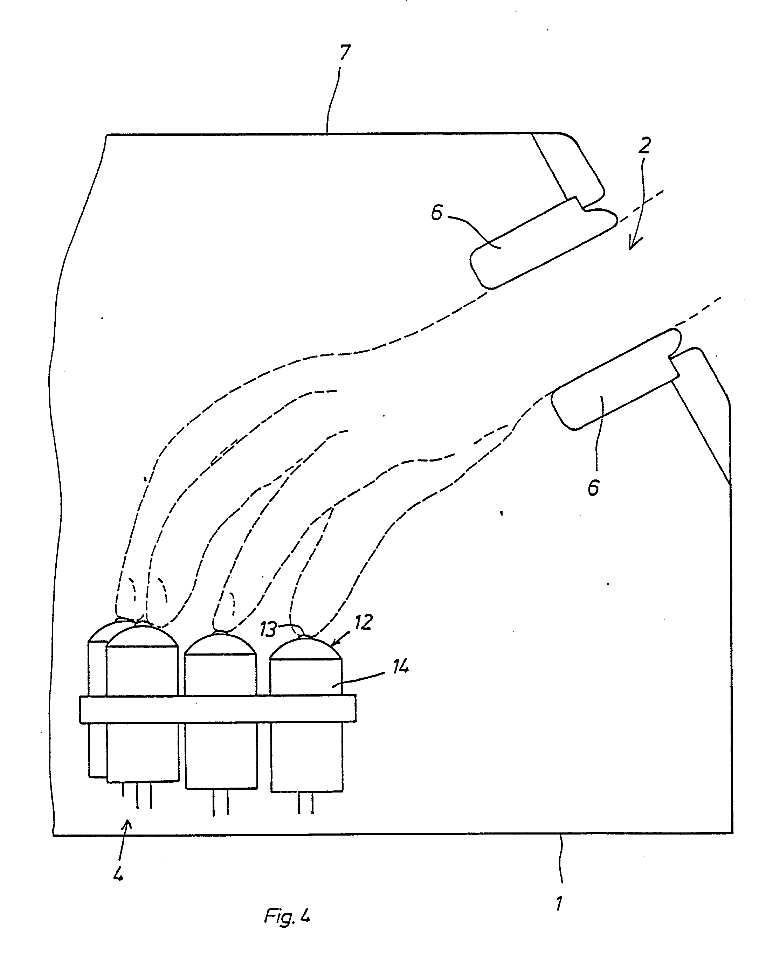

Zur Positionierung und zur Erfassung der Lage der Finger der zu reinigenden Hand weist die Kontrollvorrichtung für jeden der Finger eine eigene Auflagefläche 12 auf, wie dies aus den fig. 2 bis 4 hervorgeht. Dabei sind die einzelnen Auflageflächen 12 in den anatomischen Gegebenheiten entsprechender gegenseitiger Ausrichtung angeordnet. Ihr Abstand ist im übrigen so bemessen, daß die Finger bei den Auflageflächen 12 aufliegenden und diese abdeckenden Fingerkuppen gespreizt sind.

Jede der Auflageflächen 12 ist mit einer optoelektronischen Sensor 13 versehen, der bei nicht abgedeckter Auflagefläche 12 von zumindest einer, in der Zeichnung nicht näher dargestellten Strahlungsquelle beleuchtet ist. Die Strahlungsquelle bildet daher mit jedem der Sensoren 13 eine Art Lichtschranke, die durch jeweils einen Finger unterbrochen werden kann. Aus den Ausgangssignalen der Sensoren 13 bei entsprechend dem Reinigungsvorgang vorgeschriebener Abdeckung wird das Freigabesignal für den Reinigungsvorgang erzeugt.

Jede der Auflageflächen 12 ist jeweils von der Stirnfläche eines vertikal angeordneten Auflagekörpers 14 gebildet. Die Auflagekörpers 14 gebildet. Die Auflagekörper 14 weisen im einzelnen zylindrische Gestalt auf und sind in einer Bohrung eines die Auflagekörper 14 haltenden Verbindungsstegs 15 angeordnet. Die Auflagefläche 12 selbst ist konvex geformt, wobei in deren Scheitelbereich jeweils der Sensor 13 kuppelförmig hervorsteht. Dadurch wird eine nur punktuelle Anlage der Fingerkuppe an der Auflagefläche 12 erreicht, so daß die Reinigungswirkung im Anlagebereich praktisch nicht beeinträchtigt wird. Um den Reinigungseffekt in diesem Bereich dennoch zu erhöhen, münden in der Auflagefläche 12 zwei sich diametral gegenüberstehende Düsen 16. Die Düsenkanäle 17 verlaufen dabei in Richtung zu ihrer Mündungsöffnung schräg zur Zylinderachse des Auflagekörpers 14 hin, wodurch die Reinigungsflüssigkeit bevorzugt in den Bereich der Fingeranlage gerichtet ist.

Der Auflagekörper 14 ist im wesentlichen quer zu seiner Achse zweigeteilt, wobei der obere, die Auflagefläche 12 tragende Teil 14.1 an seinem der Auflagefläche 12 abgewandten unteren Rand einen Ringkragen 14.2 aufweist. Ein am unteren Teil 14.3 des Auflagekörpers 14 koaxial vorstehendes Zylinderstück 14.4 wird von diesem Ringkragen 14.2 dichtend umgriffen. Im Zylinderstück 14.4 ist eine Axialbohrung für die elektrische Zuleitung 18 zum Sensor 13 vorgesehen, während im Ringkragen 14.2 axial verlaufende Verbindungskanäle 19 an die Düsenkanäle 17 anschließen. Diese Verbindungskanäle 19 stehen an der Stirnseite des Ringkragens 14.2 mit einer oder mehreren Zuführleitungen 20 am unteren Teil 14.3 des Auflagekörpers 14 in Verbindung. Durch diese Ausbildung des Auflagekörpers 14 ist einerseits eine vollständige Trennung des Gas- bzw. Flüssigkeitssystems von dem elektrischen System gewährleistet, dennoch ist es ohne weiteres möglich, beispielsweise den Sensor 13 leicht auszuwechseln.

Wie sich aus der Fig. 2 ergibt, sind jeweils etwa mittig zwischen den Auflageflächen 12 weitere optoelektronische Sensoren 21 vorgesehen. Der von diesen Sensoren 21 registrierte Lichteinfall stellt einerseits sicher, daß die Finger der zu reinigenden Hand auch tatsächlich gespreizt sind, so daß die gewünschte Reinigungswirkung auch zwischen den Fingern gegeben ist. Darüber hinaus signalisieren diese Sensoren 21 die Funktionsfähigkeit der Strahlungsquelle. Wie sich aus der Fig. 2 weiter ergibt, sind die Sensoren 21 an dem Verbindungssteg 15 angeordnet und gegenüber der Verbindungslinie der in den Auflageflächen 12 vorgesehenen Sensoren 13 zum Handflächenbereich hin verlagert.

Die Kontrollvorrichtung 4 und/oder die Steuereinrichtung 5 kann im übrigen so ausgebildet sein, daß auch personenbezogene Besonderheiten, wie beispielsweise das Fehlen eines oder mehrerer Finger berücksichtigt werden, so daß selbst dann ein ordnungsgemäßer Waschvorgang ablaufen kann.

## Patentansprüche

1. Waschautomat zur Reinigung von Gegenständen, insbes. auch von Händen, mit einem im wesentlichen allseits geschlossenen Gehäuse (1) mit zumindest einer Öffnung (2) zur Einführung des zu reinigenden Gegenstands, ferner mit einer oder mehreren, im Innern des Gehäuses angeordneten, den Gegenstand mit flüssigen und/oder gasförmigen Reinigungsmedien beaufschlagenden, entsprechend ausrichtbaren Düsen (3), sowie mit einer ebenfalls im Innern des Gehäuses angeordneten, auf Form und Lage des Gegenstands im Gehäuse ansprechenden Kontrollvorrichtung (4), die ein Freigabesignal für den Reinigungsvorgang erzeugt, wenn der Gegenstand die für den Reinigungsvorgang vorgesehene Form bzw. Gestalt aufweist und/oder vorgeschriebene Lage bzw. Ausrichtung im Gehäuse einnimmt, wobei zur Positionierung und zur anschließenden Erfassung der Lage der Finger einer zu reinigenden Hand die Kontrollvorrichtung (4) für jeden der Finger eine eigene Auflagefläche (12) aufweist, wobei die einzelnen Auflageflächen (12) in den anatomischen Gegebenheiten entsprechender gegenseitiger Ausrichtung angeordnet sind und ihr Abstand so bemessen ist, daß die mit ihren Kuppen auf den Auflageflächen (12) aufliegenden Finger gespreizt sind und mit ihren Kuppen die Auflagefläche (12) abdecken, daß ferner jede der Auflageflächen (12) mit einem optoelektronischen Sensor (13) versehen ist, der bei nicht abgedeckter Auflagefläche (12) von zumindest einer Strahlungsquelle beleuchtet ist und daß aus den Ausgangssignalen der Sensoren (13) bei entsprechend dem Reinigungsvorgang vorgeschriebener Abdeckung das Freigabesignal erzeugt wird.

2. Waschautomat nach Anspruch 1, dadurch gekennzeichnet, daß jede der Auflageflächen (12) jeweils von der Stirnfläche eines vertikal angeordneten Auflagekörpers (14) gebildet ist.

3. Waschautomat nach Anspruch 2, dadurch gekennzeichnet, daß der Auflagekörper (14) zylindrische Gestalt aufweist und in einer Bohrung eines die Auflagekörper (14) haltenden, horizontal verlaufenden Verbindungsstegs (15) angeordnet ist.

4. Waschautomat nach den Ansprüchen 2 und 3, dadurch gekennzeichnet, daß die Auflagefläche (12) konvex geformt ist und in deren Scheitelbereich der Sensor (13) kuppelförmig hervorsteht.

5. Waschautomat nach den Ansprüchen 1 bis 4, dadurch gekennzeichnet, daß in der Auflagefläche (12) zumindest eine der Düsen (16) zur Abgabe des Reinigungsmediums mündet.

6. Waschautomat nach Anspruch 5, dadurch gekennzeichnet, daß in der Auflagefläche (12) zwei sich diametral gegenüberstehende Düsen (16) münden.

7. Waschautomat nach den Ansprüchen 5 und 6, dadurch gekennzeichnet, daß der Düsenkanal (17) in Richtung zu seiner Mündungsöffnung schräg zur Zylinderachse des Auflagekörpers (14) hin gerichtet verläuft.

8. Waschautomat nach den Ansprüchen 5 bis 7, dadurch gekennzeichnet, daß der Auflagekörper (14) im wesentlichen quer zu seiner Achse zweigeteilt ist, wobei der obere, die Auflagefläche (12) tragende Teil (14.1) an seinem der Auflagefläche (12) abgewandten unteren Rand einen Ringkragen (14.2) aufweist, der ein am unteren Teil (14.3) des Auflagekörpers (14) koaxial vorstehendes Zylinderstück (14.4) dichtend umgreift, wobei im Zylinderstück (14.4) eine Axialbohrung für die elektrische Zuleitung (18) zum Sensor (13) vorgesehen ist und im Ringkragen (14.2) axial verlaufende Verbindungskanäle (19) an die Düsenkanäle (17) anschließen und an der Stirnseite des Ringkragens (14.2) mit einer oder mehreren Zuführleitungen (20) am unteren Teil (14.3) des Auflagekörpers (14) in Verbindung stehen.

9. Waschautomat nach den Ansprüchen 1 bis 8, dadurch gekennzeichnet, daß jeweils etwa mittig zwischen den Auflageflächen (12) weitere optoelektronische Sensoren (21) vorgesehen sind.

10. Waschautomat nach Anspruch 9, dadurch gekennzeichnet, daß die die Fingerzwischenräume abtastenden Sensoren (21) an dem Verbindungssteg angeordnet und gegenüber der Verbindungslinie der in den Auflageflächen vorgesehenen Sensoren (13) in Richtung zur Handfläche hin verlagert sind.

**Claims**

1. Automatic washing apparatus for cleaning objects, including in particular hands, having a housing (1) substantially closed on all sides with at least one aperture (2) into which the object to be cleaned is inserted, and having one or more suitably alignable nozzles (3) which are disposed inside the housing and which act upon the object with cleaning agents in the form of fluid and/or gas, as well as having a control device (4) also disposed inside the housing, the control device responding to the form and position of the object in the housing and generating a release signal for the cleaning process if the object has the form or shape intended for the cleaning process and/or assumes the prescribed position or alignment in the housing, the control device (4) having an individual contact face (12) for each of the fingers for the purpose of positioning and subsequently determining the position of the fingers of a hand to be cleaned, the individual contact faces (12) being arranged so as to be aligned according to the anatomical arrangement and being spaced in such a manner that the fingers bearing with their tips on the contact faces (12) are spread, and further that each contact face (12) is provided with an opto-electronic sensor (13), which is irradiated by at least one source of radiation when the contact face (12) is not covered and that the release signal is generated from the output signals of the sensors (13) when the contact faces (12) are covered as specified for the cleaning process.

2. Automatic washing apparatus according to Claim 1, characterised in that each of the contact faces (12) is formed in each case by the end face of a vertically-disposed contact body (14).

3. Automatic washing apparatus according to Claim 2, characterised in that the contact body (14) has a cylindrical shape and is disposed in a bore of a horizontally-extending connecting web (15) holding the contact body (14).

4. Automatic washing apparatus according to Claims 2 and 3, characterised in that the contact face (12) is convex and the sensor (13) projects at the crown thereof in the form of a dome.

5. Automatic washing apparatus according to Claims 1 to 4, characterised in that, in the contact face (12), at least one of the nozzles (16) opens out for discharging the cleaning agent.

6. Automatic washing apparatus according to Claim 5, characterised in that, in the nozzle, two diametrically opposite nozzles (16) open out.

7. Automatic washing apparatus according to Claims 5 and 6, characterised in that the nozzle channel (17) extends towards its opening in an oblique manner with respect to the cylinder axis of the contact body (14).

8. Automatic washing apparatus according to Claims 5 to 7, characterised in that that the contact body (14) is divided into two substantially transversely with respect to its axis, the upper part (14, 1), which carries the contact face (12), having on its lower edge remote from the contact face (12), an annular collar (14, 2) enclosing in a sealing-tight manner a cylindrical member (14, 4), which projects coaxially on the lower part of the contact body (14), an axial bore being provided in the cylindrical member (14, 4) for the electrical supply line to the sensor (13), the said axial bore joining connecting channels (19), which extend axially in the annular collar (14, 2), to the nozzle channels (17) and being connected at the end face of the annular colar (14, 2) to one or more supply lines (20) in the lower part (14, 3) of the contact body (14).

9. Automatic washing apparatus according to Claims 1 to 8, characterised in that in each case, further opto-electronic sensors (21) are provided approximately centrally between the contact faces (12).

10. Automatic washing apparatus according to Claim 9, characterised in that the sensors (21) scanning the distances between the fingers are disposed on the connecting web and are displaced towards the hand surface with respect to the connecting line of the sensors (13) provided in the contact faces.

**Revendications**

1. Laveuse automatique pour nettoyer des objets, en particulier aussi les mains, avec un boîtier (1) fermé sensiblement de tous les côtés et comportant au moins une ouverture (2) pour l'introduction de l'objet à nettoyer, avec une ou plusieurs buses (3) disposées à l'intérieur du boîtier, envoyant vers l'objet des détergents liquides et/ou gazeux et orientables en conséquence, ainsi qu'avec un dispositif de contrôle (4) également disposé à l'intérieur du boîtier et répondant à la forme et à la position de l'objet dans le boîtier, qui délivre un signal de libération pour le processus de nettoyage lorsque l'objet présente la forme et respectivement le contour prévu pour le processus de nettoyage et/ou se trouve à l'intérieur du boîtier dans la position ou l'orientation prescrite, le dispositif de contrôle (4) comprenant, pour le positionnement et la détermination consécutive de la position des doigts d'une main à nettoyer, une surface d'appui (12) séparée pour chacun des doigts, les différentes surfaces d'appui (12) étant orientées les unes par rapport aux autres en fonction de critères anatomiques et leur espacement étant choisi de telle façon que les doigts reposant avec leurs bouts sur les surfaces d'appui (12) sont écartés et recouvrent avec leurs bouts lesdites surfaces d'appui (12), que, en plus, chacune des surfaces d'appui (12) est munie d'un capteur opto-électronique (13) qui, lorsque la surface d'appui (12) n'est pas recouverte, est éclairé par au moins une source de rayonnement, et que le signal de libération est produit à partir des signaux de sortie des capteurs (13) lorsque le recouvrement correspond à celui prescrit pour l'opération de nettoyage.

2. Laveuse automatique selon la revendication 1, caractérisée par le fait que chacune des surfaces d'appui (12) est constituée respectivement par la face frontale d'un corps d'appui (14) disposé verticalement.

3. Laveuse automatique selon la revendication 2, caractérisée par le fait que le corps d'appui (14) présente une forme cylindrique et qu'il est placé dans un alésage d'une barrette de jonction (15) supportant les corps d'appui (14) et s'étendant dans le sens horizontal.

4. Laveuse automatique selon l'une des revendications 2 et 3, caractérisée par le fait que la surface d'appui (12) présente une forme convexe et que le capteur (13) dépasse, en forme de calotte, de la région du sommet de ladite surface d'appui.

5. Laveuse automatique selon l'une quelconque des revendications 1 à 4, caractérisée par le fait qu'au moins l'une des buses (16) pour la distribution du détergent débouche dans la surface d'appui (12).

6. Laveuse automatique selon la revendication 5, caractérisée par le fait que deux buses (16) diamétralement opposées débouchent dans la surface d'appui (12).

7. Laveuse automatique selon l'une des revendications 5 et 6, caractérisée par le fait que le canal de buse (17) s'étend, en direction de son embouchure, en oblique par rapport à l'axe du cylindre du corps d'appui (14).

8. Laveuse automatique selon l'une quelconque des revendications 5 à 7, caractérisée par le fait que le corps d'appui (14) est divisé en deux parties, à savoir sensiblement perpendiculairement à son axe, la partie supérieure (14.1) portant la surface d'appui (12) présentant sur son bord inférieur opposé à la surface d'appui (12) un collet annulaire (14.2) qui entoure de manière étanche un élément cylindrique (14.4) lequel dépasse coaxialement de la partie inférieure (14.3) du corps d'appui (14), un alésage axial étant prévu dans l'élément cylindrique (14.4) pour le conducteur électrique (18) menant au capteur (13), et des canaux de jonction (19) s'étendant axialement dans le collet annulaire (14.2) étant raccordés aux canaux de buses (17) et communiquant, à la face frontale du collet annulaire (14.2), avec un ou plusieurs conduits d'arrivée (20) dans la partie inférieure (14.3) du corps d'appui (14).

9. Laveuse automatique selon l'une quelconque des revendications 1 à 8, caractérisée par le fait que des capteurs opto-électroniques (21) supplémentaires sont respectivement prévus à peu près au milieu entre les surfaces d'appui (12).

10. Laveuse automatique selon la revendication 9, caractérisée par le fait que les capteurs (21) explorant les intervalles entre les doigts sont placés sur la barrette de jonction et décalés, en direction de la face de la main, par rapport à la ligne de liaison des capteurs (13) prévus dans les surfaces d'appui.

*Fig.1*

Fig. 2

0 229 203

Fig. 3

Fig. 4